# EUROPÄISCHE PATENTSCHRIFT

(11) **EP 0 951 939 B1**
(45) Veröffentlichungstag und Bekanntmachung des Hinweises auf die Patenterteilung: **07.12.2005**
(21) Anmeldenummer: 99107798.3
(22) Anmeldetag: 20.04.1999
(51) Int. Cl.: B01L 11/00, G01N 33/52, G01N 27/64

(54) **Vorratsbehältnis für analytische Hilfsmittel**
Storage container for analytical test elements
Dispositif de stockage d'éléments d'analyse

(30) Priorität: 24.04.1998 DE 19818359; 25.11.1998 DE 19854316
(43) Veröffentlichungstag der Anmeldung: 27.10.1999
(73) Patentinhaber: Roche Diagnostics GmbH, 68305 Mannheim (DE)
(72) Erfinder: Sacherer, Klaus-Dieter, 67281 Kirchheim (DE)

(56) Entgegenhaltungen:
- EP-A- 0 732 590
- EP-A- 0 738 666
- US-A- 5 332 549
- US-A- 5 720 924

## Beschreibung

Die Erfindung betrifft ein Vorratsbehältnis aus einem starren Material für zwei oder mehrere analytische Hilfsmittel, bei dem diese einzeln in Kammern untergebracht werden können, die sich zueinander in einer regelmäßigen geometrischen Anordnung befinden, wobei jede der Kammern zumindest zwei gegenüberliegende, durch eine Folie verschlossene Öffnungen aufweist. Die Erfindung betrifft weiterhin ein System zur Bevorratung von analytischen Hilfsmitteln enthaltend ein erfindungsgemäßes Vorratsbehältnis und zwei oder mehrere analytische Hilfsmittel.

Für die chemische und biochemische Analyse von festen und flüssigen Probenmaterialien haben sich in den dafür spezialisierten Labors, aber insbesondere auch für den Einsatz außerhalb fester Labors trägergebundene Schnelltests etabliert. Basierend auf einer eigens entwickelten Trockenchemie sind solche trägergebundenen Schnelltests, trotz der oftmals komplexen Reaktionen unter Beteiligung empfindlicher Reagenzien, einfach und unkompliziert, selbst von Laien, durchzuführen. Prominentestes Beispiel für trägergebundene Schnelltests sind Teststreifen für die Bestimmung des Blutglukosegehaltes bei Diabetikern. Ebenso bekannt sind Ein- oder Mehrfelderteststreifen für die Urinanalytik und diverse Indikatorpapiere. Da neben Schnelltesten in Streifenform (Teststreifen) auch andere Formen von trägergebundenen Schnelltests existieren, spricht man allgemeiner von "analytischen Testelementen".

Trockenchemische, trägergebundene Schnelltests sind für den Verkauf an den Endverbraucher meist mehrfach verpackt. Die Schnelltest befinden sich meist in einer ersten, sie direkt umgebenden Verpackung (Primärverpackung), welche wiederum in einer weiteren Verpackung (Umverpackung, Sekundärverpackung) steckt, die zusätzlich zur Primärverpackung meist Handhabungshinweise für die Schnelltests in Form von Packungsbeilagen enthält. Die Primärverpackungen sind so konzipiert, daß sie die wesentlichen Aufgaben zum Erhalt der Funktion der chemischen und biochemischen Bestandteile auf dem Testelement während einer längeren Lagerzeit erfüllen. Diese Aufgaben sind vor allem Schutz vor der Einwirkung von Lichtstrahlen, Schutz vor dem Zutritt von Luftfeuchtigkeit, Schmutz, Keimen und Staub, sowie Schutz vor mechanischer Beeinträchtigung der Testelemente.

Eine der am häufigsten anzutreffenden Formen der Primärverpackung ist die Darbietung von lose eingeschütteten Testelementen in Aluminium- oder Kunststoffröhren, die durch einen aufzudrückenden oder aufzuschraubenden Stopfen verschlossen sind. Die oben skizzierten Aufgaben der Primärverpackung werden durch diese Röhrenverpackungen befriedigend gelöst. Aufgrund der umständlichen manuellen Entnahme der einzelnen Testelemente aus der Primärverpackung scheinen diese nicht mehr zeitgemäß, weshalb alternative Verpackungskonzepte entwickelt wurden. Diese bieten neben den oben genannten Eigenschaften zudem die Möglichkeit, die Testelemente einzeln automatisch aus der Verpackung zu entnehmen und direkt einem Meßgerät, welches zur Messung und anschließenden Auswertung der Testergebnisse dient, zur Verfügung zu stellen.

EP-A 0 622 119 beschreibt Bevorratungssysteme aus starren, wasserdampfundurchlässigen Materialien für streifenförmige Testelemente, bei denen die Testelemente einzeln in folienversiegelten Kammern aufbewahrt werden (Einzelversiegelung). Die Kammern für die Teststreifen - wie streifenförmige Testelemente auch bezeichnet werden - besitzen die Form von Röhren mit rechteckigem Querschnitt und sind geometrisch regelmäßig zueinander ausgerichtet, so daß sich für das Bevorratungssystem beispielsweise die Form eines im wesentlichen rechteckigen Magazins oder Klappetuis, beide mit zueinander parallelen, in einer Linie nebeneinanderliegenden Kammern, oder eines länglichen Zylinders bzw. einer flachen, kreisförmigen Scheibe mit radial um eine zentrale Achse gruppierten Kammern ergibt. Die Testelemente können sowohl manuell als auch durch eine mechanische Vorrichtung aus dem Bevorratungssystem entnommen werden, wobei die im Vorratsbehältnis verbleibenden Testelemente durch die Einzelversiegelung weiterhin geschützt sind. Außerdem beschreibt EP-A 0 622 119 die Möglichkeit, Trockenmittel wie z. B. Kieselgele und Molekularsiebe, innerhalb der Kammern für die Teststreifen bereitzustellen, um Restfeuchte aufzunehmen, die durch den Herstellprozeß der Teststreifen bedingt ist oder die trotz der Versiegelung und der Verwendung von wasserdampfundurchlässigen Materialien in die Kammern eingedrungen ist. Auf einer der Außenseiten des Bevorratungssystems kann ein Datenträger, wie z. B. ein Etikett in lesbarer Schrift, ein Strichcodeetikett oder ein Magnetstreifen, angebracht sein, auf dem chargenspezifische Daten und gegebenenfalls weitere Informationen zu den Testelementen im System gespeichert und abrufbar sind. Ein Teil der in EP-A 0 622 119 beschriebenen Vorratsbehältnisse für Testelemente sind für den Einsatz in entsprechend gestalteten Meßsystemen, bestehend im wesentlichen aus Meßgerät, Vorratsbehältnis und Testelementen, geeignet.

EP-A 0 732 590 und US 5,489,414 beschreiben runde, scheibenförmige Vorratsbehältnisse für Testelemente, die für den Einsatz in kompakten Meßgeräten, beispielsweise zur Blutzuckerselbstkontrolle bei Diabetikern, geeignet sind. Die Testelemente sind hier sternförmig in einer Ebene um den Scheibenmittelpunkt angeordnet und einzeln in Blistern, wie sie beispielsweise zur Einzelverpackung von Tabletten bekannt sind, schmutz- und feuchtigkeitsdicht eingesiegelt. Im Vorratsbehältnis nach EP-A 0 732 590 ist für jedes Testelement ein separates Blister für ein Trockenmittel vorgesehen, wobei Trockenmittelblister und Testelementeblister miteinander verbunden sind, so daß eine effektive Entfeuchtung des Testelementeblisters gewährleistet ist.

Aus US 5,510,266 und EP-A 0 738 666 sind zylindrische, in Spritzgußverfahren aus Kunststoffen hergestellte Testelementemagazine bekannt, bei denen die einzelnen Testelemente ähnlich den Patronen in einer Revolvertrommel in von der Grundfläche des Zylinders zur gegenüberliegenden Deckelfläche durchgehenden Kammern angeordnet sind. Wie bereits weiter oben zu EP-A 0 622 119 beschrieben sind die Testelemente hier in parallel zueinander verlaufenden, radial um eine zentrale Längsachse angeordneten, länglichen, röhrenförmigen Kammern einzeln eingesiegelt, wozu die kreisförmigen Grund- bzw. Deckelflächen des zylindrischen Vorratsbehältnisses mit Folien, wie z. B. Aluminiumfolie, versiegelt sind. Zur Entnahme der Testelemente aus dem Magazin wird mit einem Stößel eine der Siegelfolien durchstochen und das zu entnehmende Testelement durch die gegenüberliegende Siegelfolie aus seiner Kammer geschoben und so seinem Bestimmungszweck zugeführt. In Analogie zu EP-A 0 732 590 ist für jede Testelementekammer eine separate Trockenmittelkammer vorgesehen, die mit der Testelementekammer über einen Kanal verbunden ist, so daß die Testelementekammer durch das Trockenmittel zuverlässig entfeuchtet werden kann. Auch die Testelementemagazine aus US 5,510,266 und EP-A 0 738 666 sind prinzipiell für den Einsatz in kompakten Meßgeräten konzipiert.

Die im Stand der Technik beschriebenen Vorratsbehältnisse weisen den Nachteil auf, daß die Testelemente nicht optimal vor Umwelteinflüssen, insbesondere mechanischen Einflüssen geschützt sind. Die in EP-A 0 732 590 und US 5,489,414 beschriebenen Blisterverpackungen für Testelemente sind aus verhältnismäßig dünnen Kunststoffolien hergestellt und bieten naturgemäß nur unzureichenden Schutz der Testelemente vor mechanischer Beschädigung, beispielsweise durch unbeabsichtigtes Drücken oder Knicken der Verpackung. Die Testelementevorratsbehältnisse aus US 5,510,266 und EP-A 0 738 666 bieten hier besseren Schutz, da sie aus starren, festen Materialien hergestellt werden, die zumindest Druck- und Knickbelastungen besser standhalten als Blisterverpackungen. Die mechanischen Schwachstellen des in US 5,510,266 und EP-A 0 738 666 offenbarten trommelförmigen Verpackungstyps sind jedoch die Siegelfolien, mit denen die Grund- und Deckelfläche der zylindrischen Trommel verschlossen sind, um so abgeschlossene Testelementekammern zu erzeugen. Diese Siegelfolien sind - damit sie bei der Testelemententnahme leicht durchstochen werden können - in der Regel aus dünnen Folien, beispielsweise Aluminiumfolien, hergestellt und werden bei unbeabsichtigtem Fallenlassen oder unvorsichtigem Aufsetzen der Verpackung auf eine Unterlage leicht verletzt. Da bereits geringfügige Öffnungen in den Siegelfolien ein Eindringen von Staub, Keimen und Luftfeuchtigkeit in die Testelementekammern ermöglicht, können die durch die Verpackung zu schützenden Testelemente dadurch ernsthaften Schaden nehmen.

Ein weiterer Nachteil der im Stand der Technik beschriebenen Vorratsbehältnisse liegt darin, daß die automatische Testelemententnahme, beispielsweise mit Hilfe eines Stößels, oftmals zu einem Verkanten der Testelemente in ihrer Kammer führt. Die Zuverlässigkeit dieser Systeme bei der Testelementebereitstellung ist demnach nur unzureichend.

Die beschriebenen Nachteile der Verpackungskonzepte für Testelemente bestehen im wesentlichen auch für andere analytische Hilfsmittel, beispielsweise Lanzetten oder Probennahmeelemente. Obwohl bei den letztgenannten Hilfsmitteln in der Regel keine empfindlichen Reagenzien vor Umwelteinflüssen geschützt zu werden brauchen, ist jedoch auch hier auf sterile Bedingungen in den Kammern des Vorratsbehältnisses zu achten, nicht daß die bevorrateten Elemente bei längerer Lagerung unbrauchbar werden.

Aufgabe der Erfindung ist es, die Nachteile des Standes der Technik zu beseitigen. Insbesondere ist es die Aufgabe der vorliegenden Erfindung, ein kompaktes und in hoher Stückzahl kostengünstig herstellbares Vorratsbehältnis für analytische Hilfsmittel, also z.B. Testelemente, Probennahmeelemente und Lanzetten, bereitzustellen, welches die darin befindlichen analytischen Hilfsmittel sicher vor schädlichen Umwelteinflüssen, wie z.B. Licht, Feuchtigkeit oder mechanischer Einwirkung, schützen kann. Des weiteren soll das Vorratsbehältnis in ein Analysensystem, enthaltend ein kompaktes Messgerät, das Vorratsbehältnis und Testelemente, integrierbar sein und eine zuverlässige, d.h. fehlerfreie Entnahme der analytischen Hilfsmittel, ermöglichen.

Gegenstand der Erfindung ist ein Vorratsbehältnis zur Verwendung in einem kompakten Messgerät, wobei das Vorratsbehältnis aus einen starren Material für zwei oder mehrere analytische Hilfsmittel enthaltend separate Kammern, in denen jeweils höchstens ein Hilfsmittel untergebracht werden kann, wobei die Kammern sich zueinander in einer regelmäßigen geometrischen Anordnung befinden und jede der Kammern zumindest zwei gegenüberliegende, jeweils durch eine Folie verschlossene Öffnungen aufweist, dadurch gekennzeichnet, daß jede Kammer zur Fixierung der Position der analytischen Hilfsmittel in der Kammer aufweist.

Unter dem Begriff "analytisches Hilfsmittel" sollen analytische Testelemente, Küvetten, Pipetten oder Lanzetten verstanden werden. Vorzugsweise handelt es sich um analytische Testelemente oder Lanzetten, besonders bevorzugt um analytische Testelemente. Analytische Testelemente im hier benutzten Sinn sind visuell oder apparativ-optisch auswertbare Teststreifen, elektrochemische Sensoren und dergleichen mehr. Da derartige analytische Hilfsmittel im Stand der Technik umfassend beschrieben und dem Fachmann in einer Vielzahl von Ausführungsformen geläufig sind, erübrigt sich hier eine detaillierte Beschreibung. Beispielsweise sei auf folgende Dokumente verwiesen: Deutsche Patentanmeldung Aktenzeichen 197 53 847.9, EP-A 0 138 152, EP-A 0 821 234; EP-A 0 821 233, EP-A 0 630 609 EP-A 0 565 970 und WO 97/02487.

Das erfindungsgemäße Vorratsbehältnis entspricht bezüglich Form, Funktion und Materialien weitgehend dem Stand der Technik. Insbesondere seien hier genannt: EP-A 0 622 119, EP-A 0 738 666 und US 5,510,266. Auf diese Dokumente wird hier ausdrücklich Bezug genommen. Besonders bevorzugt hat das erfindungsgemäße Vorratsbehältnis die Form einer im wesentlichen zylindrischen, länglichen Trommel, in der die Kammern zur Aufnahme der analytischen Hilfsmittel sternförmig um die Längsachse angeordnet sind. Die Höhe der Trommel richtet sich im wesentlichen nach der Länge der unterzubringenden analytischen Hilfsmittel. Die Grund- und Deckelflächen des zylindrischen Behältnisses enthalten dabei die Öffnungen der Kammern, welche mit Folie dicht verschlossen sind. Vorzugsweise werden sämtliche Öffnungen einer Fläche individuell und unabhängig voneinander verschlossen, wobei jedoch nur ein Stück Folie verwendet wird. Durch das individuelle und unabhängige Verschließen ist dafür gesorgt, daß beim Öffnen einer Kammer die Siegelfolien für die verbleibenden Kammern nicht beschädigt werden.

Vorzugsweise ist der Grundkörper des erfindungsgemäßen Vorratsbehältnisses aus einem starren, spritzgußfähigen Kunststoff hersgestellt, wie z. B. Polyethylen oder Polypropylen. Die Folie zum Abdichten der Kammeröffnungen, die auch als Siegelfolie bezeichnet wird, besteht vorzugsweise aus Aluminium oder einem Aluminium-Kunststofflaminat und ist über an sich bekannte Verfahren wie Verschweißen oder Verkleben mit dem Kunststoffgrundkörper des Vorratsbehältnisses dicht verbunden. Vorzugsweise wird die Siegelfolie mittels Schmelzkleber auf dem Spritzgußgrundkörper befestigt. Um zu verhindern, daß Klebstoffreste in die Hilfsmittelkammern hinein- oder über den Rand des Vorratsbehältnisses herausragen, können in einer bevorzugten Ausführungsform des erfindungsgemäßen Vorratsbehältnisses an einzelnen oder sämtlichen betroffenen Kanten Bereiche zur Aufnahme von Klebstoffresten vorgesehen sein. Beispielsweise kann an allen betroffenen Kanten eine umlaufende Vertiefung zur Aufnahme von Klebstoffresten vorhanden sein.

Je Kammer für analytische Hilfsmittel ist in einer bevorzugten Ausführungsform des erfindungsgemäßen Vorratsbehältnisses ein individueller Trockenmittelvorrat vorgesehen, der vorzugsweise in einer separaten Trockenmittelkammer untergebracht ist. Als Trockenmittel können prinzipiell sämtliche als Feststoffe oder pastöse Massen erhältlichen Trockenmittel, insbesondere Kieselgel, Molekularsieb und ähnliches mehr, eingesetzt werden. Je nach Menge des Trockenmittels, welches zur Entfeuchtung der Hilfsmittelkammer erforderlich ist, wird die Trockenmittelkammer dimensioniert. Trockenmittelkammer und Hilfsmittelkammer stehen in einem Gasaustausch ermöglichenden Kontakt. Sie sind bevorzugt durch einen Kanal miteinander verbunden, der einen Luftaustausch der Kammern und somit ein Entfeuchten der Hilfsmittelkammer ermöglicht. Vorzugsweise ist der Kanal so dimensioniert und geometrisch gestaltet, daß das Trockenmittel, beispielsweise Kieselgel oder Molekularsieb, nicht in die Hilfsmittelkammer eindringen kann. Gegebenenfalls ist die Größe der Trockenmittelpartikel entsprechend zu wählen.

Vorzugsweise enthält die Trockenmittelkammer zwei Öffnungen, von denen die eine dem Befüllen der Kammer mit Trockenmittel dient (Befüllöffnung) und die andere den Gasaustausch ermöglichenden Kontakt mit der Kammer für das analytische Hilfsmittel herstellt (Kanalöf fnung). Während die Kanalöffnung jederzeit geöffnet bleiben muß, um ein Entfeuchten der Hilfsmittelkammer zu ermöglichen, kann die Befüllöffnung der Trockenmittelkammer nach dem Einfüllen des Trockenmittels verschlossen werden. Auf diese Weise kann einem unbeabsichtigten Entweichen des Trockenmittels aus der Kammer, beispielsweise bei nachfolgenden Herstell- oder Befüllschritten des erfindungsgemäßen Vorratsbehältnisses, vorgebeugt werden. Das Verschließen der Befüllöffnung der Trockenmittelkammer kann bevorzugt durch Abdecken bspw. mit Karton, Papier, Kunststoff- oder Metallfolie erfolgen. Weiterhin bevorzugt ist die Abdeckung der Befüllöffnung durch einen Kunststoff- oder Klebstoffpfropfen. Besonders bevorzugt wird die Befüllöffnung der Trockenmittelkammer dadurch verschlossen, daß mit Hilfe eines geeigneten Werkzeugs Spritzgußmaterial aus dem Randbereich der Öffnung verstemmt, d. h. in die Öffnung verdrängt wird und so eine Abdeckung der Öffnung formt.

Ein erfindungswesentlicher Unterschied zu den im Stand der Technik bekannten Vorratsbehältnissen für analytische Hilfsmittel liegt darin, daß in jeder Kammer, die zur Aufnahme eines einzelnen analytischen Hilfsmittels dient, Mittel zur Fixierung der Position des analytischen Hilfsmittels in der Kammer vorgesehen sind. Die Fixierung des analytischen Hilfsmittels in der Kammer hat sich überraschenderweise als vorteilhaft herausgestellt, da hierdurch ein Verletzen der Folie, welche die Kammern einzeln verschließt und somit auch das Eindringen von Staub, Schmutz, Feuchtigkeit und Keimen unterbindet, verhindert wird. Beim unbeabsichtigten Fallenlassen, Schütteln oder Stoßen des Vorratsbehältnisses kann es mit den im Stand der Technik beschriebenen Vorratsbehältnissen, bei denen die analytischen Hilfsmittel lose und somit beweglich in den Kammern liegen, zu einer Perforation der Siegelfolie durch das analytische Hilfsmittel kommen. Ein sicheres Abdichten der einzelnen Kammern während Fertigung, Lagerung, Transport und Benutzung des Vorratsbehältnisses und somit ein zuverlässiger Schutz der analytischen Hilfsmittel in den Kammern ist mit diesen Behältnissen nicht zu gewährleisten. Mit der erfindungsgemäßen Einführung von Mitteln zur Fixierung der Position der analytischen Hilfsmittel in der jeweiligen Kammer wird dieses Problem gelöst. Die Fixierung des analytischen Hilfsmittels in seiner Kammer verhindert weitestgehend, daß die Siegelfolie der Kammer durch das analytische Hilfsmittel unbeabsichtigt perforiert wird.

Als Mittel zur Fixierung der analytischen Hilfsmittel in einer festen Position in der Kammer sind erfindungsgemäß verschiedene Ausgestaltungsformen möglich. Neben der stabilen Fixierung der Position der analytischen Hilfsmittel in der Kammer müssen diese Mittel jedoch erlauben, daß das analytische Hilfsmittel leicht in die Kammer zu befüllen und aus dieser bei ihrer Verwendung wieder zu entnehmen ist.

Als bevorzugtes Mittel zur Fixierung der analytischen Hilfsmittel in einer festen Position in der Kammer hat sich herausgestellt, die Kammer teilweise zu verengen, vorzugsweise in demjenigen Bereich der Kammer, welcher der Entnahmeöffnung des analytischen Hilfsmittels gegenüberliegt. Die Verengung kann kontinuierlich, z. B. konisch, oder stufenweise ausgestaltet sein und das analytische Hilfsmittel von einer oder mehreren Seiten fixieren. Die Verengung der Kammer kann die Kammerwand oder -wände auf ihrer gesamten Fläche betreffen. Es ist jedoch auch möglich, daß die Kammer zur Verengung eine oder mehrere Erhebungen auf der Kammerwand oder den Kammerwänden, die ins Kammerinnere weisen, aufweist. Die Erhebungen können gleich oder unterschiedlich geformt sein und beispielsweise in Form von Kuppeln, Stegen, Wülsten, Rippen oder ähnlichem vorliegen. Die Erhebungen in der Kammerwand führen dazu, daß das analytische Hilfsmittel zur Fixierung der Position in der Kammer nur teilweise berührt wird und erlauben somit eine Optimierung der Kräfte, mit denen die Fixierung erzielt wird.

Erfindungsgemäß hat es sich als besonders bevorzugt herausgestellt, die analytischen Hilfsmittel sowohl über eine teilweise konische Verengung der Kammerwand als auch über eine oder mehrere Erhebungen in der Kammerwand in der Kammer zu fixieren. Ganz besonders bevorzugt dienen als Erhebungen drei Stege, die sich auf zwei gegenüberliegenden Kammerwänden befinden und die das analytische Hilfsmittel in der Kammer leicht verbiegen und so über die erzeugte Biegespannung in seiner Position fixieren. Die Verbiegung darf selbstverständlich nicht zu einer Beschädigung des analytischen Hilfsmittels oder einer Beeinträchtigung der Funktion des analytischen Hilfsmittels führen.

Weiterhin hat es sich als erfindungsgemäß bevorzugt herausgestellt, daß je Kammer im erfindungsgemäßen Vorratsbehältnis nur eine der mindestens zwei Öffnungen der Kammer für die Befüllung und Entnahme der analytischen Hilfsmittel geeignet ist. Nur eine dieser zwei Öffnungen ist demnach groß genug, um das analytische Hilfsmittel durch sie hindurch zu entnehmen bzw. um das analytische Hilfsmittel beim Befüllen durch sie hindurch in die Kammer einzubringen. Im Folgenden soll diese Eigenschaft der Öffnung kurz als "durchlässig für analytische Hilfsmittel" bezeichnet werden.

Bei den Vorratsbehältnissen gemäß dem Stand der Technik (insbesondere EP-A 0 738 666 und US 5,510,266) sind sowohl die Öffnungen in der Bodenfläche als auch die Öffnungen in der Deckelfläche für die im Vorratsbehältnis enthaltenen analytischen Hilfsmittel durchlässig. Beim Befüllen des Vorratsbehältnisses mit analytischen Hilfsmitteln gemäß dem genannten Stand der Technik wird zunächst eine Fläche des Vorratsbehältnisses mit einer Folie versiegelt und anschließend eine Anzahl von analytischen Hilfsmitteln in die dafür vorgesehenen Kammern eingefüllt. Schließlich wird die noch offene Fläche ebenfalls mit einer Folie versiegelt. Dieses Verfahren weist die Nachteile auf, daß beim Befüllen der Kammern mit den analytischen Hilfsmitteln die erste Folie verletzt werden kann und daß zwei Herstellschritte zum Versiegeln der Kammern erforderlich sind. Bei dem erfindungsgemäß bevorzugten Vorratsbehältnis, bei dem jeweils nur eine der mindestens zwei Öffnungen der Kammern für die analytischen Hilfsmittel durchlässig ist, treten diese Nachteile nicht auf. Das Befüllen der Kammern mit analytischen Hilfsmitteln kann erfolgen, bevor eine der Öffnungen durch eine Siegelfolie verschlossen ist, da eine der Öffnungen der Kammer für die analytischen Hilfsmittel nicht durchlässig ist und somit als Boden für die Kammer dienen kann, auf dem das eingebrachte analytische Hilfsmittel aufliegt. Die Verletzungsgefahr für die Folie, die bei diesem Prozeßschritt noch nicht anwesend sein muß, wird dadurch minimiert. Weiterhin kann der Prozeß der Versiegelung der Kammern durch Folien von beiden Öffnungsseiten gleichzeitig erfolgen. Zudem kann die Siegelfolie, die auf derjenigen Seite des erfindungsgemäßen Vorratsbehältnisses angebracht ist, auf der sich die für die analytischen Hilfsmittel undurchlässigen Kammeröffnungen befinden, grundsätzlich nicht von innen durch das sich in der Kammer befindliche analytische Hilfsmittel verletzt oder durchstoßen werden, was die Zuverlässigkeit des erfindungsgemäßen Vorratsbehältnisses erhöht.

Die Entnahme der analytischen Hilfsmittel aus dem erfindungsgemäßen Vorratsbehältnis erfolgt durch Herausschieben des analytischen Hilfsmittels aus der Kammer, vorzugsweise mit Hilfe eine Stößels. Für die bevorzugte Ausführungsform des erfindungsgemäßen Vorratsbehältnisses, bei dem eine der beiden Öffnungen der Kammer für das analytische Hilfsmittel nicht durchlässig ist, ist es bevorzugt, daß diese Öffnung für einen Stößel durchlässig ist, der das analytische Hilfsmittel aus dem Vorratsbehältnis schieben kann. Ganz besonders ist es bevorzugt, daß jede Kammer eine Führungsnut für den Stößel enthält. Diese hält den Stößel und das in der Kammer befindliche analytische Hilfsmittel während des Ausstoßvorgangs in einer exakt definierten gegenseitigen Lagebeziehung, so daß ein Verkanten oder Aneinandervorbeigleiten von Stößel und analytischem Hilfsmittel verhindert wird.

Da die Folien, die zum Verschließen der Öffnungen der Kammern des erfindungsgemäßen Vorratsbehältnisses dienen, zur Entnahme des analytischen Hilfsmittels aus der Kammer von diesem durchtrennbar sein müssen, stellen sie naturgemäß eine potentielle mechanische Schwachstelle des erfindungsgemäßen Vorratsbehältnisses dar. Die Auswahl für Material und Dicke der Folie ist dadurch eingeschränkt, daß ein Durchtrennen der Siegelfolie mit Hilfe des analytischen Hilfsmittels in der Kammer bei Druck des Stößels auf das analytische Hilfsmittel möglich sein muß. Zudem darf das analytische Hilfsmittel beim Durchtrennen der Folie nicht beschädigt werden. Um diese Siegelfolien beispielsweise beim Abstellen des Vorratsbehältnisses auf eine flache Unterlage zu schützen, hat es sich erfindungsgemäß als vorteilhaft herausgestellt, auf den mit Folien verschlossenen Flächen des Vorratsbehältnisses Erhebungen vorzusehen, die beim Abstellen des Vorratsbehältnisses auf eine flache Unterlage einen direkten Kontakt zwischen Folie und Unterlage verhindern. Diese Erhebungen können als umlaufender, dünner Randsteg an der äußeren Peripherie des erfindungsgemäßen Vorratsbehältnisses ausgestaltet sein. Als ebenso vorteilhaft haben sich Erhebungen im Zentrum der mit Folie versiegelten Flächen des erfindungsgemäßen Vorratsbehältnisses heraugestellt. Die Erhebung kann jede beliebige Form annehmen, beispielsweise Stege oder eine Vielzahl regelmäßig verteilter Noppen. Die Höhe der Erhebung richtet sich im wesentlichen nach der Dicke der verwendeten Siegelfolie. Um erfindungsgemäß wirksam zu sein, muß die Erhebung mindestens die Dicke der Siegelfolie plus der Dicke einer eventuell vorhandenen Kleberschicht zur Fixierung der Siegelfolie auf dem erfindungsgemäßen Vorratsbehältnis aufweisen. Vorzugsweise ragt die Erhebung jedoch mindestens 300 bis 400 µm über die Oberfläche der Siegelfolie heraus. Vorzugsweise werden die Erhebungen nicht mit der Siegelfolie überzogen, sondern die Bereiche, in denen Erhebungen vorhanden sind, vom Versiegeln ausgespart. Vorteilhafterweise werden in diesem Fall Siegelfolien verwendet, die vor dem Aufbringen auf den Grundkörper des Vorratsbehältnisses mit einer entsprechenden Aussparung versehen wurden. Das Aufbringen einer derartigen Siegelfolie erfordert naturgemäß ein genaues Positionieren der Siegelfolie relativ zum Vorratsbehältnisgrundkörper.

Als besonders vorteilhaft hat es sich herausgestellt, daß zumindest eine der Flächen des erfmdungsgemäßen Vorratsbehältnisses, welche mit einer Siegelfolie versehen ist, nicht eben sondern in Form eines nach innen gerichteten Konus ausgestaltet ist. Vorzugsweise ist dies die Fläche, aus der die analytischen Hilfsmittel beim Einsatz eines Stößels aus ihrer Kammer herausgeschoben werden. Selbstverständlich kann auch die gegenüberliegende Fläche dieses Merkmal aufweisen oder beide Flächen, die mit Folie versiegelt sind. Die konische Form der Fläche besitzt den Vorteil, daß die Siegelfolie vor unbeabsichtigter Beschädigung geschützt ist, da nur der äußere Rand auf einer ebenen Fläche aufliegen kann. Zudem wird die Kraft, die zum Durchtrennen der Folie erforderlich ist, durch diese Geometrie reduziert. Vorzugsweise weist der Konus einen Neigungswinkel bezogen auf die ebene Fläche von 1° bis 45°, besonders bevorzugt von 1° bis 10°, ganz besonders bevorzugt von 5° auf.

Für die Aufnahme des erfindungsgemäßen Vorratsbehältnisses in ein Meßgerät und zum automatisierten Entnehmen einzelner analytischer Hilfsmittel können entsprechende Mittel im oder am Vorratsbehältnis vorgesehen sein. Wichtig erscheint in diesem Zusammenhang vor allem die genaue Positionierbarkeit des Vorratsbehältnisses relativ zu funktionalen Bestandteilen eines Meßgeräts, und hierbei insbesondere zum Stößel für die Hilfsmittelentnahme. In einer bevorzugten Ausführungsform enthält das erfindungsgemäße Vorratsbehältnis daher eine zentrale Bohrung, in die ein passender Führungsstift des Meßgeräts eingreifen kann. Des weiteren kann in der Bohrung oder von dieser getrennt eine Kerbe oder ein Zahnkranz vorhanden sein, in den eine entsprechende Antriebsvorrichtung des Meßgeräts eingreifen kann, um das Vorratsbehältnis in eine begünstigte Entnahmeposition zu bringen.

In die zentrale Bohrung des Vorratsbehältnisses greift in einem entsprechenden Meßgerät ein Führungsstift ein, der das Vorratsbehältnis in der richtigen Position für die Entnahme der Hilfsmittel hält. Beispielsweise am Rand der zentralen Bohrung kann sich ein Antriebszahnkranz befinden, in den ein entsprechend geformtes Gegenstück beim Einsatz des Vorratsbehältnisses in ein Meßgerät eingreifen kann und mit dessen Hilfe das Vorratsbehältnis im Meßgerät rotiert werden kann. Durch die Rotation des Vorratsbehältnisses im Meßgerät kann das Vorratsbehältnis in entsprechend vordefinierte Positionen gebracht werden, so daß mit Hilfe eines Stößels aus dem Meßgerät die Testelemententnahme und das Bereitstellen von Testelementen für Meßvorgänge ermöglicht wird.

Ein weiterer Gegenstand der Erfindung ist ein System zur Bevorratung von analytischen Hilfsmitteln enthaltend ein erfindungsgemäßes Vorratsbehältnis und zwei oder mehrere analytische Hilfsmittel.

Das erfindungsgemäße System enthält ein erfindungsgemäßes Vorratsbehältnis gemäß obiger Beschreibung. In dem Vorratsbehältnis befinden sich mindestens zwei, vorzugsweise 10 bis 20 analytische Hilfsmittel, jeweils einzeln in eine Kammer eingesiegelt. Besonders bevorzugt handelt es sich bei den analytischen Hilfsmitteln um Testelemente für die Analyse von Flüssigkeiten, z. B. diagnostische Teststreifen, oder um Lanzetten, wobei Testelemente ganz besonders bevorzugt sind. Selbstverständlich ist es erfindungsgemäß auch möglich, mehrere Arten von analytischen Hilfsmitteln, beispielsweise also Testelemente und Lanzetten, in jeweils eigene Kammern unterzubringen.

Das erfindungsgemäße System kann zudem ein kompaktes Meßgerät enthalten, welches das erfindungsgemäße Vorratsbehältnis mit den darin enthaltenen analytischen Hilfsmitteln, vorzugsweise Testelementen, in sich aufnehmen kann und die analytischen Hilfsmittel aus dem Vorratsbehältnis zu entnehmen in der Lage ist. Die analytischen Hilfsmittel werden dabei dem Meßgerät zur Verfügung gestellt, um die gewünschte Analyse damit durchzuführen.

Schließlich ist Gegenstand der Erfindung ein System zur Bevorratung von analytischen Hilfsmitteln enthaltend ein oder mehrere erfindungsgemäße Vorratsbehältnisse und je Vorratsbehältnis zwei oder mehrere analytische Hilfsmittel, wobei die Vorratsbehältnisse in einem Behältnis enthalten sind.

Zur zusätzlichen Sicherung der analytischen Hilfsmittel und der erfindungsgemäßen Vorratsbehältnisse vor schädlichen Umwelteinflüssen, insbesondere Feuchtigkeit, Licht und mechanischer Beanspruchung, können diese nochmals in ein sie umgebendes Behältnis, z. B. eine mit einem Stopfen verschließbare Metall- oder Kunststoffröhre, verpackt werden. Dieses das oder die Vorratsbehältnis umgebende Behältnis kann vorzugsweise ein weiteres Trockenmittel enthalten und so die Lagerstabilität der analytischen Hilfsmittel, die sich in den erfindungsgemäßen Vorratsbehältnissen befinden, erhöhen.

Die Vorteile der Erfindung können wie folgt zusammengefaßt werden:
◆ Durch die Fixierung der analytischen Hilfsmittel in den Kammern des Vorratsbehältnisses wird ein Schutz der Siegelfolie bei unbeabsichtigter mechanischer Beanspruchung, beispielsweise beim Fallenlassen, Stoßen oder Schütteln, erzielt.
◆ Die Fixierung der analytischen Hilfsmittel in den Kammern des Vorratsbehältnisses führt zu einer genauen Positionierung der Hilfsmittel relativ zu einem Stößel, mit dessen Hilfe sie aus der Kammer geschoben werden können. Ein Vorbeigleiten des Stößels am Hilfsmittel beim Ausschieben aus dem Vorratsbehältnis wird dadurch verhindert.
◆ Die Fixierung der analytischen Hilfsmittel in den Kammern des Vorratsbehältnisses dient außerdem als Führung für die Hilfsmittel beim Ausschieben aus dem Vorratsbehältnis. Die Gefahr des Verkantens der Hilfsmittel bei diesem Vorgang wird dadurch minimiert.
◆ Da die gegenüberliegenden Kammeröffnungen vorzugsweise unterschiedlich gestaltet und so nur auf einer Seite für die analytischen Hilfsmittel durchlässig sind, ist die Siegelfolie auf der Fläche, die für die Hilfsmittel nicht durchlässig ist, beim Fallenlassen, Stoßen oder Schütteln des Behältnisses nicht durch den Inhalt der Kammern gefährdet; zudem vereinfacht sich die Fertigung, da die Versiegelung beider Flächen, welche die Öffnungen der Kammern enthalten, in einem Arbeitsschritt möglich ist.
◆ Durch die spezielle Gestaltung derjenigen Flächen des erfindungsgemäßen Vorratsbehältnisses, die mit einer Siegelfolie verschlossen sind, wird ein Beschädigen der Folie beim Abstellen des Vorratsbehältnisses auf eine ebene Unterlage vermieden. Unter der speziellen Gestaltung wird unter anderem verstanden, daß Erhebungen auf den Flächen und/oder konisch nach innen gerichtete Flächen vorgesehen sind.
◆ Eine geschützte Siegelfolie trägt wiederum zum Schutz der analytischen Hilfsmittel in den Kammern des Vorratsbehältnisses bei.
◆ Zudem kann der Grundkörper des erfindungsgemäßen Vorratsbehältnisses kostengünstig durch Spritzguß eines Kunststoffs hergestellt werden.

Die Erfindung wird durch die nachfolgenden Zeichnungen näher erläutert.
Figur 1 zeigt eine Seitenansicht einer bevorzugten Ausführungsform des erfindungsgemäßen Vorratsbehältnisses.
Figur 2 zeigt schematisch eine Aufsicht auf die kreisförmige Grundfläche (Deckel) der bevorzugten Ausführungsform des erfindungsgemäßen Vorratsbehältnisses aus Figur 1, bei dem die Siegelfolie vollständig entfernt wurde.
Figur 3 zeigt schematisch eine Aufsicht auf die kreisförmige Grundfläche (Boden) der bevorzugten Ausführungsform des erfindungsgemäßen Vorratsbehältnisses aus Figur 1, bei dem die Siegelfolie vollständig entfernt wurde.
Figur 4 zeigt einen schematischen Längsschnitt durch eine bevorzugte Ausführungsform des erfindungsgemäßen Vorratsbehältnisses aus Figur 1.
Figur 5 zeigt einen schematischen Querschnitt durch eine bevorzugte Ausführungsform des erfindungsgemäßen Vorratsbehältnisses aus Figur 1.
Figur 6 zeigt schematisch eine Detailvergrößerung einer Testelementekammer, wie sie in Figur 2 zu sehen ist, in Aufsicht.
Figur 7 zeigt einen schematischen Längsschnitt durch eine weitere bevorzugte Ausführungsform eines erfindungsgemäßen Vorratsbehältnisses.
Figur 8 zeigt in einer schematischen Detailvergrößerung einen Querschnitt entlang der Linie B-B' durch zwei Kammern der in Figur 7 abgebildeten Ausführungsform des erfindungsgemäßen Vorratsbehältnisses.
Figur 9 zeigt schematisch ein erfindungsgemäß bevorzugtes System enthaltend drei erfindungsgemäße Vorratsbehältnisse für Testelemente und ein Behältnis für diese Vorratsbehältnisse in Form einer mit einem Stopfen verschließbaren Röhre.

Die Ziffern in den Figuren bedeuten:
- 1: Vorratsbehältnis
- 2: Testelement
- 3: Testelementekammer
- 4: konisch angeschrägte Oberseite des Vorratsbehältnisses 1
- 5: Siegelfolie der Oberseite 4
- 6: Erhebung auf der konisch angeschrägten Oberseite 4 des Vorratsbehältnisses 1
- 7: Trockenmittelkammer
- 8: plane Unterseite des Vorratsbehältnisses 1
- 9: Erhebung auf der planen Unterseite 8 des Vorratsbehältnisses 1
- 10: zentrale Bohrung mit Antriebszahnkranz
- 11: Siegelfolie der Unterseite 8
- 12: Öffnung für Stößel
- 13: Öffnung für Testelemententnahme
- 14: Öffnung für Trockenmittelbefüllung
- 15: Kammerwand der Testelementekammer 3
- 16: Verengung der Testelementekammer 3
- 17: Führungsnut für Stößel
- 18: stegförmige Erhebung in der Kammerwand 15
- 19: Lanzette
- 20: Lanzettenkammer
- 21: Lanzettenkörper
- 22: röhrenförmiges Behältnis für drei Vorratsbehältnisse 1
- 23: Stopfen für röhrenförmiges Behältnis 22

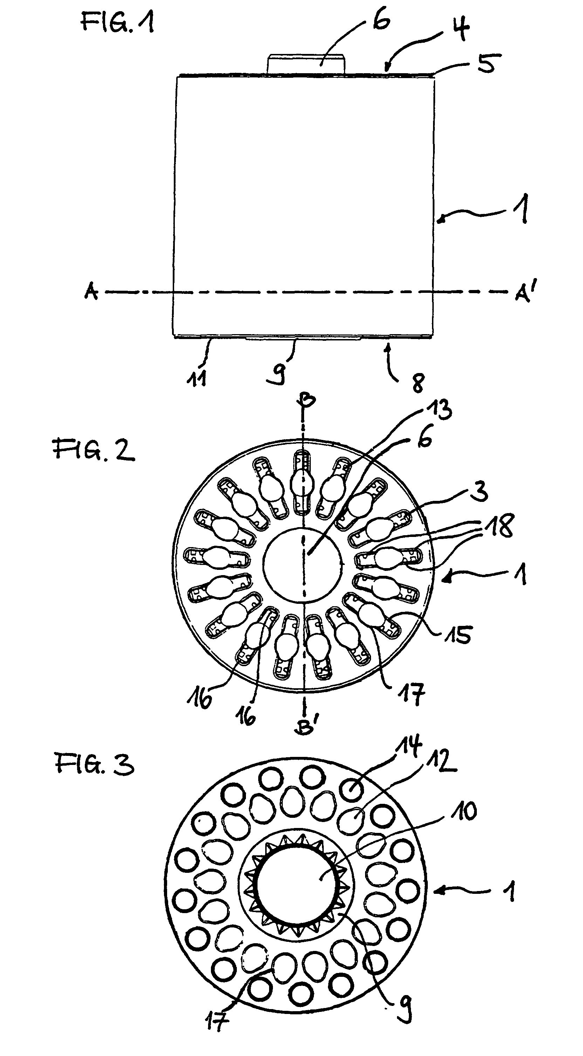

In Figur 1 ist eine besonders bevorzugte Ausführungsform des erfindungsgemäßen Vorratsbehältnisses (1), hier zur Bevorratung von analytischen Testelementen, in einer Seitenansicht dargestellt. Das Vorratsbehältnis (1) ist im wesentlichen wie eine zylindrische Trommel geformt, die eine kreisförmige, konisch angeschrägte Oberseite (4) besitzt und eine im wesentliche plane Unterseite (8). Die Oberseite (4) ist dabei diejenige Seite, aus der die Testelemente entnommen werden können. Die Unterseite (8) ist diejenige Seite, durch die ein Stößel zum Herausschieben der Testelemente in das Vorratsbehältnis (1) eindringen kann. Das dargestellte Vorratsbehältnis (1) ist vorzugsweise aus einem starren, spritzgußfähigen Kunststoff, wie zum Beispiel Polyethylen oder Polypropylen, gefertigt. Die konisch angeschrägte Oberseite (4) und die plane Unterseite (8) sind zum Schutz der im Vorratsbehältnis (1) enthaltenen analytischen Testelemente mit Siegelfolien (5,11) versehen. Diese Siegelfolien (5,11) können mit dem Spritzgußgrundkörper des Vorratsbehältnisses (1) verklebt oder verschweißt sein. Zum Schutz der Siegelfolien (5, 11) sind sowohl auf der Unterseite (8) des Vorratsbehältnisses (1) als auch auf der Oberseite (4) Erhebungen (6,9) vorgesehen. Diese Erhebungen (6,9) sind vorzugsweise Bestandteil des Spritzgußgrundkörpers des Vorratsbehältnisses (1). Sie sorgen dafür, daß die Siegelfolien (5,11) beim Abstellen des Vorratsbehältnisses (1) auf eine ebene Unterlage nicht beschädigt werden. Die Siegelfolien (5, 11) besitzen im Bereich der Erhebungen (6,9) Aussparungen, so daß die Erhebungen (6,9) nicht von den Siegelfolien (5, 11) bedeckt sind.

In Figur 2 ist eine Aufsicht auf die konisch angeschrägte Oberseite (4) des Vorratsbehältnisses (1) dargestellt. Man erkennt deutlich eine Vielzahl von Testelementekammern (3), die sternförmig um die Erhebung (6) der konisch angeschrägten Oberseite (4) des Vorratsbehältnisses (1) angeordnet sind. Die Testelementekammern (3) enthalten auf der zur konisch angeschrägten Oberseite (4) des Vorratsbehältnisses (1) zugewandten Seite die Öffnung zur Testelemententnahme (13).

Im Inneren der Testelementekammer (3) sind Mittel zum Fixieren des Testelementes in der Testelementekammer (3) vorgesehen. Zum einen ist eine Verengung (16) der Testelementekammer (3) enthalten, die von zwei gegenüberliegenden Seiten ein in der Kammer enthaltenes Testelement fixieren kann. Zum anderen befinden sich in jeder Testelementekammer (3) stegförmige Erhebungen (18) in der Kammerwand (15). Zudem enthält die Kammerwand (15) eine Führungsnut (17) für einen Stößel.

In Figur 3 ist eine Aufsicht auf die plane Unterseite (8) des Vorratsbehältnisses (1) abgebildet, wobei auch hier wie in Figur 2 die Siegelfolie abgezogen ist. Um eine zentrale Bohrung (10) mit Antriebszahnkranz, die von einer Erhebung (9) umgeben ist, sind in dieser Ansicht die Öffnungen (12) für einen Stößel und die Öffnung (14) für die Trockenmittelbefüllung sichtbar. Auf derjenigen Seite der Testelementekammer (3), die der planen Unterseite (8) zugewandt ist, ist eine Öffnung (12) für einen Stößel vorgesehen, mit dessen Hilfe Die Testelemente aus der Testelementekammer (3) geschoben werden können. Die Öffnungen (12) für den Stößel sind mit den Führungsnuten (17) für den Stößel in Verbindung.

Die Trockenmittelkammern sind über einen in Figur 3 nicht sichtbaren Kanal mit den Testelementekammern verbunden. Die Dimension des Kanals ist dabei so gewählt, daß einzelne Trockenmittelpartikel nicht von der Trockenmittelkammer in die Testelementekammer gelangen können. Ein Gasaustausch zwischen Trockenmittelkammer und Testelementekammer ist dabei natürlich zu gewährleisten.

In Figur 4 ist ein schematischer Längsschnitt entlang der Linie B-B' des erfindungsgemäß bevorzugten Vorratsbehältnisses (1) aus Figur 2 abgebildet. Der Querschnitt verdeutlicht insbesondere die Lage und Form einer Testelementekammer (3), einer Trockenmittelkammer (7) sowie der zentralen Bohrung (10) mit Antriebszahnkranz. Desweiteren ist dem Querschnitt aus Figur 4 deutlich zu entnehmen, wie die Oberseite (4) des Vorratsbehältnisses (1) konisch angeschrägt ist. Weiterhin ist ein Testelement (2) schematisch abgebildet, um dessen Lage in der Testelementekammer (3) zu verdeutlichen.

Durch einen Stößel, der durch die Siegelfolie (11) der Unterseite (8) und durch die dafür vorgesehene Öffnung (12) in die Testelementekammer (3) eindringt, kann das Testelement (2) nach oben aus der Öffnung (13) und durch die Siegelfolie (5) der Oberseite (4) aus dem Vorratsbehältnis (1) entnommen werden. Das Testelement (2) wird im Vorratsbehältnis (1) durch eine Verengung (16) sowie durch stegförmige Erhebungen in der Kammerwand (15) in der Testelementekammer (3) in seiner Position fixiert. Ein unbeabsichtigtes Durchstoßen der Siegelfolie (5) der Oberseite des Vorratsbehältnisses (1) wird damit weitgehend verhindert. Ein Durchstoßen der Siegelfolie (11) der Unterseite (8) des Vorratsbehältnisses (1) mit dem Testelement (2) wird dadurch verhindert, daß der Boden (8) des Vorratsbehältnisses (1) im Bereich der Testelementekammer (3) lediglich eine Öffnung (12) für einen Stößel enthält, die für das Testelement (2) nicht durchlässig ist.

Die zentrale Bohrung (10) ist für die Aufnahme des Vorratsbehältnisses (1) in ein Meßgerät gedacht. In die zentrale Bohrung (10) greift in einem entsprechenden Meßgerät ein Führungsstift ein, der das Vorratsbehältnis (1) in der richtigen Position hält. Die Erhebung (6) auf der konisch angeschrägten Oberseite (4) des Vorratsbehältnisses (1) kann neben der oben beschriebenen Funktion des Schutzes der Siegelfolie (5) der Oberseite (4) des Vorratsbehältnisses (1) ebenfalls zur Stabilisierung der Lage des Vorratsbehältnisses (1) in einem Meßgerät dienen. Die Erhebung (6) kann dort beispielsweise in eine entsprechende Vertiefung oder Aussparung eingreifen.

Am unteren Rand der zentralen Bohrung (10) befindet sich ein Antriebszahnkranz, in den ein entsprechend geformtes Gegenstück beim Einsatz des Vorratsbehältnisses (1) in ein Meßgerät eingreifen kann und mit dessen Hilfe das Vorratsbehältnis (1) im Meßgerät rotiert werden kann. Durch die Rotation des Vorratsbehältnisses (1) im Meßgerät kann das Vorratsbehältnis (1) in entsprechend vordefinierte Positionen gebracht werden, so daß mit Hilfe eines Stößels aus dem Meßgerät die Testelemententnahme und das Bereitstellen von Testelementen für Meßvorgänge ermöglicht wird.

In der hier beschriebenen, besonders bevorzugten Ausführungsform des erfindungsgemäßen Vorratsbehältnisses befindet sich diametral gegenüber jeder Testelementekammer (3) eine Trockenmittelkammer (7), die über eine Öffnung (14) mit einem üblichen Trockenmittel, wie z. B. Kieselgel oder Molekularsieb, befüllt werden kann. Jede Trockenmittelkammer (7) ist einer unmittelbar benachbarten Testelementekammer (3) zugeordnet und mit dieser über einen Kanal verbunden, der einen Luftaustausch zwischen Trockenmittelkammer (7) und Testelementekammer (3) ermöglicht.

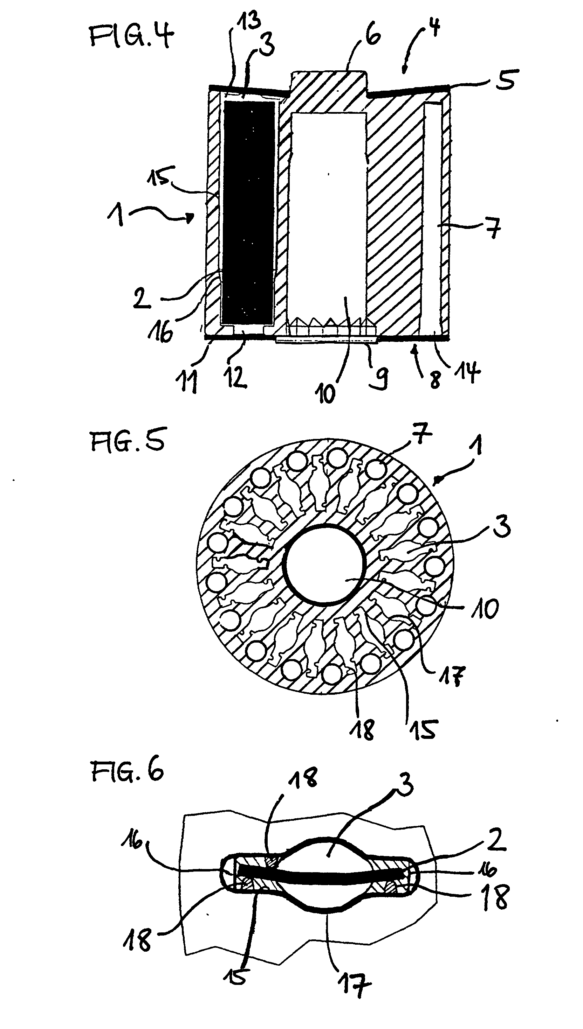

In Figur 5 ist ein Querschnitt entlang der Linie A-A' des erfindungsgemäß besonders bevorzugten Vorratsbehältnisses (1) aus Figur 1 abgebildet. In dieser Abbildung sind besonders deutlich die stegförmigen Erhebungen (18) in der Kammerwand (15) der Testelementekammer (3) zu sehen. Je Testelementekammer (3) sind drei solcher Erhebungen vorgesehen.

Figur 6 zeigt anhand eines vergrößerten Detailausschnittes einer Testelementekammer (3), wie sie in Figur 2 zu sehen ist, wie ein Testelement (2) mit Hilfe der stegförmigen Erhebungen (18) in der Kammerwand (15) der Testelementekammer (3) in seiner Position fixiert wird. Die stegförmigen Erhebungen (18) der Kammerwand (15) der Testelementekammer (3) sorgen für ein leichtes Durchbiegen des Testelements (2), so daß dieses aufgrund der Biegespannung in der Testelementekammer (3) fixiert ist. Zur weiteren Fixierung des Testelements (2) dienen die Verengungen (16) der Testelementekammer (3). Die Befüllung der Testelementekammer (3) mit dem Testelement (2) erfolgt durch Einschieben jeweils eines Testelements (2) in die Kammer (3).

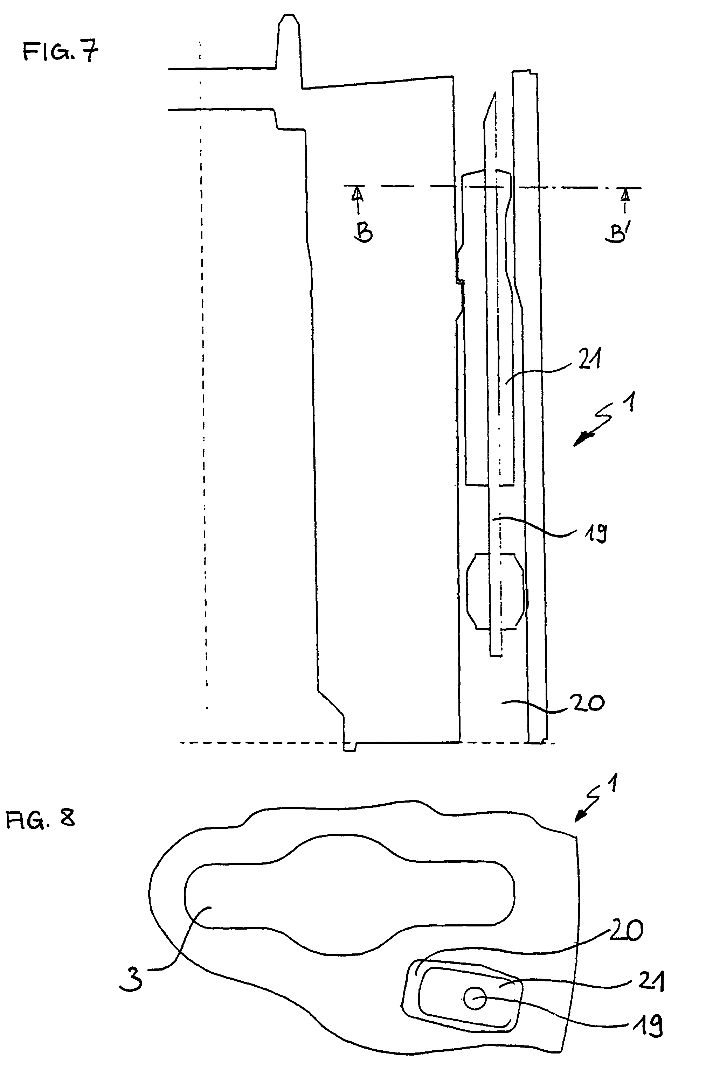

In Figur 7 ist ein schematischer Längsschnitt durch eine weitere bevorzugte Ausführungsform des erfindungsgemäßen Vorratsbehältnisses (1) abgebildet. Anders als in den bisher beschriebenen Ausführungsformen enthält die in Figur 7 abgebildete Ausführungsform als analytisches Hilfsmittel eine Lanzette (19), die in einer Lanzettenkammer (20) untergebracht ist. Die Lanzette (19) ist teilweise von einem aus Kunststoff gefertigten Lanzettenkörper (21) umgeben.

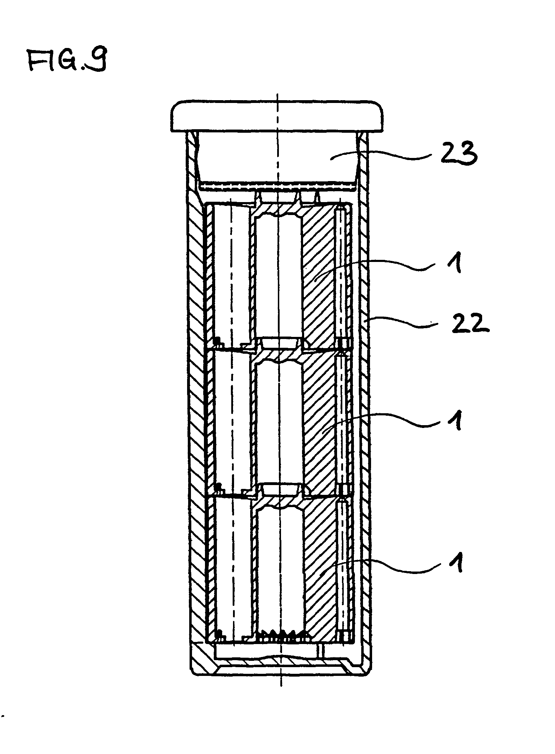

Wie aus Figur 8 deutlich wird, kann das erfindungsgemäße Vorratsbehältnis (1) neben einer Testelementekammer (3) ebenfalls eine Lanzettenkammer (20) enthalten. Die geometrische Anordnung von Testelementekammer (3) und Lanzettenkammer (20) kann in analoger Weise wie die geometrische Anordnung der Testelementekammer (3) und der Trockenmittelkammer (7), wie sie beispielsweise aus Figur 5 ersichtlich ist, erfolgen. Prinzipiell ist es möglich, neben Testelementekammern (3) und Lanzettenkammern (20) Trockenmittelkammern vorzusehen, die beispielsweise über einen Kanal mit jeweils einer Testelementekammer (3) in einem Gasaustausch ermöglichendem Kontakt stehen. Es ist jedoch auch möglich, für die in Figur 8 gezeigte besonders bevorzugte Ausführungsform auf separate Trockenmittelkammern zu verzichten. Beispielsweise ist es möglich, die Innenwände der Testelementekammern (3) aus einem Trockenmittel enthaltenden Kunststoff zu fertigen. Ebenfalls ist es möglich, den Lanzettenkörper (21) aus einem trockenmittelhaltigem Kunststoff zu fertigen. Im letztgenannten Fall ist es erforderlich, den Gasaustausch zwischen jeweils einer Testelementekammer (3) und einer Lanzettenkammer (20) beispielsweise durch einen sie verbindenden Kanal zu ermöglichen.

In Figur 9 ist schematisch ein erfindungsgemäß bevorzugtes System abgebildet, welches in diesem bevorzugten Fall aus drei erfindungsgemäß bevorzugten Vorratsbehältnissen (1) und einem mit einem Stopfen (23) verschließbaren, röhrenförmigen Behältnis (22) besteht. Das in Figur 9 gezeigte System dient dem Schutz der erfindungsgemäßen Vorratsbehältnisse (1), beispielsweise während der Lagerung und dem Transport zum Endverbraucher. Das röhrenförmige Behältnis (22) wird vorzugsweise aus einem stabilen, licht- und feuchtigkeitsundurchlässigen Kunststoff oder Metall, beispielsweise Polyethylen, Polypropylen oder Aluminium gefertigt. Der Stopfen (23) ist vorzugsweise ebenfalls aus einem der genannten Materialien gefertigt. In der gezeigten Form wird der Stopfen (23) einfach in die Röhre (22) gedrückt und diese somit dicht verschlossen. Selbstverständlich kann die Röhre (22) aber auch über einen Schraubverschluß oder einen Klappverschluß verschlossen werden. Im röhrenförmigen Behältnis (22) kann zur Stabilisierung der in den Vorratbehältnissen (1) enthaltenen Testelemente ein weiteres Trockenmittel vorgesehen sein, welches entweder am Boden des röhrenförmigen Behältnisses (22) oder im Stopfen (23) untergebracht sein kann.

## Patentansprüche

1. Vorratsbehältnis (1) zur Verwendung in einem kompakten Meßgerät, wobei das Vorratsbehältnis (1) aus einem starren Material für zwei oder mehrere analytische Hilfsmittel (2) enthaltend separate Kammern (3), in denen jeweils höchstens ein Hilfsmittel (2) untergebracht werden kann, wobei die Kammern sich zueinander in einer regelmäßigen geometrischen Anordnung befinden und jede der Kammern (3) zumindest zwei gegenüberliegende, durch jeweils eine Folie (5, 11) verschlossene Öffnungen (12, 13) aufweist
**dadurch gekennzeichnet, daß** jede Kammer (3) Mittel zur Fixierung der Position der analytischen Hilfsmittel (2) in der Kammer (3) aufweist.

2. Vorratsbehältnis gemäß Anspruch 1, **dadurch gekennzeichnet, daß** als Mittel zur Fixierung der analytischen Hilfsmittel eine teilweise Verengung der Kammer dient.

3. Vorratsbehältnis gemäß Anspruch 2, **dadurch gekennzeichnet, daß** die Kammer zur Verengung eine oder mehrere Erhebungen in der Kammerwand, die ins Kammerinnere weisen, aufweist.

4. Vorratsbehältnis gemäß einem der Ansprüche 1 bis 3, **dadurch gekennzeichnet, daß** je Kammer nur eine der mindesten zwei Öffnungen für die analytisches Hilfsmittel durchlässig ist.

5. Vorratsbehältnis gemäß Anspruch 4, **dadurch gekennzeichnet, daß** die eine der mindestens zwei Öffnungen jeder Kammer, die für die analytischen Hilfsmittel nicht durchlässig ist, für einen Stößel durchlässig ist, der die analytischen Hilfsmittel aus dem Vorratsbehältnis schieben kann.

6. Vorratsbehältnis gemäß Anspruch 5, **dadurch gekennzeichnet, daß** jede Kammer eine Führungsnut für den Stößel enthält.

7. Vorratsbehältnis gemäß einem der Ansprüche 1 bis 6, **dadurch gekennzeichnet, daß** auf den mit Folien verschlossenen Flächen des Vorratsbehältnisses Erhebungen vorhanden sind, die beim Abstellen des Vorratsbehältnisses auf eine flache Unterlage einen direkten Kontakt zwischen Folie und Unterlage verhindern.

8. System zur Bevorratung von analytischen Hilfsmitteln enthaltend ein Vorratsbehältnis gemäß einem der Ansprüche 1 bis 7und ein kompaktes Meßgerät mit zwei oder mehrere analytische Hilfsmittel.

9. System gemäß Anspruch 8, **dadurch gekennzeichnet, daß** die analytischen Hilfsmittel Testelemente zur Analyse von Flüssigkeiten sind.

10. System gemäß Anspruch 8 oder 9, weiterhin enthaltend ein Meßgerät, welches zur Aufnahme eines oder mehrerer Vorratsbehältnisse gemäß einem der Ansprüche 1 bis 7 geeignet ist.

11. System zur Bevorratung von analytischen Hilfsmitteln enthaltend ein oder mehrere Vorratsbehältnisse gemäß einem der Ansprüche 1 bis 7 und je Vorratsbehältnis zwei oder mehrere analytische Hilfsmittel, wobei die Vorratsbehältnisse in einem Behältnis enthalten sind.

12. System gemäß Anspruch 11, **dadurch gekennzeichnet, daß** das Behältnis für die Vorratsbehältnisse ein Trockenmittel enthält.

13. Vorratsbehältnis gemäß Anspruch 1 **dadurch gekennzeichnet, daß** das analytische Hilfsmittel in der Kammer leicht verbogen wird, so daß es über eine erzeugte Biegespannung in seiner Position fixiert wird.

## Claims

1. Storage container (1) for use in a compact measuring instrument, where the storage container (1) is made of a rigid material for two or more analytical devices (2) containing separate chambers (3) in each of which at most one device (2) can be accommodated, where the chambers are in a regular geometric arrangement in relation to one another and each of the chambers (3) has at least two opposite openings (12, 13) each sealed by a foil (5, 11), **characterized in that** each chamber (3) has means for fixing the position of the analytical devices (2) in the chamber (3).

2. Storage container as claimed in claim 1, **characterized in that** a partial constriction of the chamber serves as a means for fixing the analytical devices.

3. Storage container as claimed in claim 2, **characterized in that** the chamber has one or more elevations in the chamber wall pointing towards the chamber interior for the constriction.

4. Storage container as claimed in one of the claims 1 to 3, **characterized in that** the analytical devices can pass through only one of the at least two openings in each chamber.

5. Storage container as claimed in claim 4, **characterized in that** one of the at least two openings of each chamber through which the analytical devices cannot pass allows passage of a plunger which can push the analytical devices from the storage container.

6. Storage container as claimed in claim 5, **characterized in that** each chamber contains a guide groove for the plunger.

7. Storage container as claimed in one of the claims 1 to 6, **characterized in that** elevations are present on the surfaces of the storage container that are sealed with foils which prevent direct contact between the foil and support when the storage container is placed on a flat support.

8. System for storing analytical devices containing a storage container as claimed in one of the claims 1 to 7 and a compact measuring instrument with two or several analytical devices.

9. System as claimed in claim 8, **characterized in that** the analytical devices are test elements for analysing liquids.

10. System as claimed in claim 8 or 9, additionally containing a measuring instrument which is suitable for holding one or more storage containers as claimed in one of the claims 1 to 7.

11. System for storing analytical devices containing one or more storage containers as claimed in one of the claims 1 to 7 and two or more analytical devices per storage container in which the storage containers are contained in a container.

12. System as claimed in claim 11, **characterized in that** the container for the storage containers contains a desiccant.

13. Storage container as claimed in claim 1, **characterized in that** the analytical device is slightly bent in the chamber such that it can be fixed in its position by means of a resulting bending stress.

## Revendications

1. Dispositif de stockage (1) destiné à être utilisé dans un appareil de mesure compact, ledit dispositif de stockage (1) en matériau rigide pour deux ou plusieurs éléments d'analyse (2) comprenant des chambres (3) séparées pouvant recevoir chacune, au maximum, un élément d'analyse (2), lesdites chambres se trouvant l'une par rapport à l'autre dans une disposition géométrique régulière et chacune des chambres (3) comportant au moins deux orifices (12, 13) se faisant face, obturés chacun par une feuille (5, 11),
**caractérisé en ce que** chaque chambre (3) comporte des moyens pour fixer la position des éléments d'analyse (2) dans la chambre (3).

2. Dispositif de stockage selon la revendication 1, **caractérisé en ce qu'**un rétrécissement partiel de la chambre sert de moyen pour fixer les éléments d'analyse.

3. Dispositif de stockage selon la revendication 2, **caractérisé en ce que** la chambre, pour obtenir ledit rétrécissement, comporte u ne ou plusieurs bosses dans la paroi de chambre, orientées vers l'intérieur de la chambre.

4. Dispositif de stockage selon l'une des revendications 1 à 3, **caractérisé en ce que** par chambre seul l'un desdits au moins deux orifices est perméable aux éléments d'analyse.

5. Dispositif de stockage selon la revendication 4, **caractérisé en ce que** celui desdits au moins deux orifices de chaque chambre, qui n'est pas perméable aux éléments d'analyse, est perméable à un poussoir apte à faire sortir les éléments d'analyse du dispositif de stockage.

6. Dispositif de stockage selon la revendication 5, **caractérisé en ce que** chaque chambre comporte une rainure de guidage pour le poussoir.

7. Dispositif de stockage selon l'une des revendications 1 à 6, **caractérisé en ce que** des bosses sont présentes sur les surfaces obturées par des feuilles, bosses qui au moment de déposer le dispositif de stockage sur un support plat empêchent un contact direct entre feuille et support.

8. Système de stockage de moyens d'analyse, comprenant un dispositif de stockage selon l'une des revendications 1 à 7 et un appareil de mesure compact muni de deux ou plusieurs éléments d'analyse.

9. Système selon la revendication 8, **caractérisé en ce que** les éléments d'analyse sont des éléments de test permettant l'analyse de liquides.

10. Système selon la revendication 8 ou 9, comprenant en outre un appareil de mesure apte à recevoir un ou plusieurs dispositifs de stockage selon l'une des revendications 1 à 7.

11. Système de stockage de moyens d'analyse, comprenant un ou plusieurs dispositifs de stockage selon l'une des revendications 1 à 7 et par dispositif de stockage deux ou plusieurs éléments d'analyse, les dispositifs de stockage étant contenus dans un récipient.

12. Système selon la revendication 11, **caractérisé en ce que** le récipient pour les dispositifs de stockage contient un agent dessiccateur.

13. Dispositif de stockage selon la revendication 1, **caractérisé en ce que** l'élément d'analyse est légèrement plié dans la chambre de manière à le fixer dans sa position par une contrainte de flexion engendrée.
